# EUROPÄISCHE PATENTANMELDUNG

(11) **EP 4 674 387 A2**
(43) Veröffentlichungstag der Anmeldung: **07.01.2026**
(21) Anmeldenummer: 25217697.9
(22) Anmeldetag: 04.10.2021
(51) Int. Cl.: A61F 2/30

(54) **VERFAHREN UND COMPUTERPROGRAMM ZUM ERSTELLEN VON FERTIGUNGSDATEN SOWIE VERFAHREN ZUR FERTIGUNG EINER ORTHOPÄDIETECHNISCHEN EINRICHTUNG**

(30) Priorität: 08.10.2020 DE 102020126435
(62) Teilanmeldung aus: 21786490.9
(71) Anmelder: Ottobock SE & Co. KGaA, 37115 Duderstadt (DE)
(72) Erfinder: Schmidt, Michael, 37083 Göttingen (DE); Käppel, Christian, 14052 Berlin (DE); Daur, Christian, 13125 Berlin (DE)
(74) Vertreter: Gramm, Lins & Partner Patent- und Rechtsanwälte PartGmbB

(57) **Zusammenfassung**

Die Erfindung betrifft ein Verfahren zum Erstellen von Fertigungsdaten zur Fertigung einer orthopädietechnischen Einrichtung, die unter Anwendung der erstellten Fertigungsdaten in einem automatisierten Fertigungsverfahren herstellbar ist, wobei das Verfahren die folgenden Schritte umfasst:
- Bereitstellen eines digitalen 3D-Körperteilmodells eines Körperteils in einer Datenverarbeitungsanlage,
- Bereitstellen von mindestens einem digitalen Funktionskomponentenmodell einer orthopädietechnischen Funktionskomponente in der Datenverarbeitungsanlage, die in eine orthopädietechnische Einrichtung integrierbar ist, wobei das digitale Funktionskomponentenmodell entsprechende Komponenteneigenschaften der jeweiligen orthopädietechnischen Funktionskomponente enthält,
- Generieren mindestens einer digitalen Komponentenschnittstelle mittels der Datenverarbeitungsanlage in Abhängigkeit von mindestens einer Komponenteneigenschaft wenigstens eines ausgewählten digitalen Funktionskomponentenmodells einer orthopädietechnischen Funktionskomponente, die in die orthopädietechnische Einrichtung integriert werden soll, wobei die digitalen Komponentenschnittstelle eine Aufnahme zum Anordnen der mindestens einen ausgewählten orthopädietechnischen Funktionskomponente aufweist,
- Automatisches Erstellen eines digitalen orthopädietechnischen Modells der zu fertigenden orthopädietechnischen Einrichtung basierend auf dem 3D-Körperteilmodell und der digitalen Komponentenschnittstelle zur Integration der orthopädietechnischen Funktionskomponente mittels der Datenverarbeitungsanlage, und
- Generieren der digitalen Fertigungsdaten aus dem erstellten digitalen orthopädietechnischen Modell mittels der Datenverarbeitungsanlage.

## Beschreibung

Die Erfindung betrifft ein Verfahren zum Erstellen von Fertigungsdaten zur Fertigung einer orthopädietechnischen Einrichtung, die unter Anwendung der erstellten Fertigungsdaten in einem automatisierten Fertigungsverfahren herstellbar ist. Die Erfindung betrifft ebenso ein Computerprogramm hierzu.

Die Erfindung betrifft außerdem ein Verfahren zur Fertigung einer orthopädietechnischen Einrichtung basierend auf den erstellten Fertigungsdaten.

Orthopädietechnische Einrichtungen, wie beispielsweise Orthesen oder Prothesen oder auch Exoskelette, müssen präzise und genau an die vorherrschenden Bedingungen der gehandicapten oder der zu unterstützenden Person entwickelt und angepasst werden. Hierfür sind auch heute noch eine Vielzahl von manuellen und von Hand auszuführenden Arbeitsschritten notwendig, um eine solche orthopädietechnische Einrichtung herzustellen.

In der Praxis wird hierfür aus einer Formmasse zunächst ein Negativabdruck eines Körperteils erstellt, für das die orthopädietechnische Einrichtung gedacht ist. Der Negativabdruck wird anschließend ausgegossen, beispielsweise unter Anwendung von Gips. Dieses so erstellte Körperteilmodell wird in der Regel durch einen Orthopädietechniker unter Kenntnis einer bekannten medizinischen Indikation oder einer gewünschten Unterstützungsformen angepasst bzw. leicht verändert, damit die spätere orthopädietechnische Einrichtung wie beispielsweise eine Orthese oder auch Prothese, ihren medizinischen Zweck oder ihre vorgesehene Unterstützungsformen erfüllen kann. Das so an die medizinische Indikation angepasste Modell des betreffenden Körperteils wird dabei auch orthopädietechnische Zweckform genannt.

Basierend auf der so erstellten Zweckform wird nun die orthopädietechnische Einrichtung für die gehandicapte oder zu unterstützende Person entwickelt und hergestellt, indem die orthopädietechnische Einrichtung mit ihrer Form, Geometrie und Abmessungen an die erstellte Zweckform angepasst wird.

Nachteilig hierbei ist, dass ein solcher Prozess zur Herstellung einer derartigen orthopädietechnischen Einrichtung arbeitsintensiv ist und meist mehrere Tage benötigt, bis die orthopädietechnische Einrichtung der gehandicapten Person überreicht werden kann. Grund hierfür ist die Tatsache, dass an dem gesamten Prozess mehrere unterschiedliche Personengruppen beteiligt sind, die jeweils unterschiedliche Aufgaben bei der Erstellung der orthopädietechnischen Einrichtung haben. Anpassungen, die im Laufe des Prozesses notwendig werden, benötigen somit viel Zeit, bis sich die betreffende Person bzw. Personengruppe der Aufgabe annehmen kann. Zudem ist der Prozess stark abhängig von der Erfahrung des Orthopädietechnikers und liefert bei mehrfachem Durchlaufen unterschiedliche Ergebnisse.

Aus der nachveröffentlichten 10 2019 109 781.9 ist ein Verfahren zum Erstellen von Fertigungsdaten zur automatischen Herstellung einer orthopädietechnischen Einrichtung mittels einer automatisierten Fertigungsanlage bekannt, bei dem zunächst eine digitale Zweckform bereitgestellt wird. Basierend auf der digitalen Zweckform wird ein Volumenmodell der herzustellenden orthopädietechnischen Einrichtung mittels einer Datenverarbeitungsanlage erstellt, wobei basierend auf dem digitalen Volumenmodell dann die Fertigungsdaten für die automatisierte Fertigungsanlage generiert werden.

Je nach Anwendungszweck der orthopädietechnischen Einrichtung müssen komplexe mechanische und/oder mechatronische Bauteile wie beispielsweise Hydraulik, Schaltungen, Batterien, Prozessoren, Aktuatoren, Sensoren etc. in die orthopädietechnische Einrichtung integriert werden, um der orthopädietechnischen Einrichtung zusätzliche Funktionen zu verleihen. Diese als Funktionskomponenten bezeichneten Bauteile weisen in der Regel eine komplexe Struktur auf und können auch in absehbarer Zeit nicht wirtschaftlich sinnvoll in einem automatisierten Fertigungsverfahren hergestellt bzw. gedruckt werden.

Daher muss bei der Erstellung einer orthopädietechnischen Einrichtung die spätere Integration einer oder mehrerer Funktionskomponenten im Designprozess berücksichtigt werden, was den Herstellungsprozess an sich verlangsamt und deutlich kostenintensiver werden lässt. Denn nicht nur die einfache Integration der Funktionskomponente ist dabei entscheidend, sondern auch das Zusammenspiel und Zusammenwirken der Funktionskomponente mit der orthopädietechnischen Einrichtung während des bestimmungsgemäßen Gebrauches. So muss beispielsweise sichergestellt sein, dass die integrierte Funktionskomponente genügend Bewegungsraum hat, um ihre Funktion ausführen zu können und darüber hinaus so in die orthopädietechnische Einrichtung integriert ist, dass die auftretenden Lasten abgetragen werden können und nicht zu einer Beschädigung der orthopädietechnischen Einrichtung führen. Eine solche orthopädietechnische Einrichtung kann dabei sowohl einen Großteil des späteren Gesamtsystems (Einrichtung plus Funktionskomponente) ausmachen oder die orthopädietechnische Einrichtung spielt nur eine untergeordnete Rolle (bspw. lediglich kosmetischer Natur), in die größere Funktionskomponenten integriert werden (wie bspw. Kniegelenke).

Es ist daher Aufgabe der vorliegenden Erfindung ein verbessertes Verfahren zur Herstellung einer orthopädietechnischen Einrichtung anzugeben, mit der sich prozesssicher auch Komponenten integrieren lassen.

Die Aufgabe wird mit dem Verfahren zum Erstellen von Fertigungsdaten gemäß Anspruch 1, mit dem Computerprogramm gemäß Anspruch 14 sowie dem Verfahren zur Fertigung einer orthopädietechnischen Einrichtung gemäß Anspruch 15 erfindungsgemäß gelöst. Vorteilhafte Ausgestaltungen der Erfindung finden sich in den entsprechenden Unteransprüchen.

Gemäß Anspruch 1 wird ein Verfahren zum Erstellen von Fertigungsdaten zur Fertigung einer orthopädietechnischen Einrichtung vorgeschlagen, wobei die orthopädietechnische Einrichtung unter Anwendung der erstellten Fertigungsdaten in einem automatisierten Fertigungsverfahren herstellbar ist bzw. hergestellt werden soll. Die Fertigungsdaten dienen demzufolge als Grundlage zur Ansteuerung einer automatisierten Fertigungsanlage, die das automatisierte Fertigungsverfahren zur Herstellung der orthopädietechnischen Einrichtung ausführt. Solche Fertigungsdaten können beispielsweise Computermodell sein, auf deren Basis Steuersignale zur Ansteuerung der automatisierten Fertigungsanlage zur Herstellung der orthopädietechnischen Einrichtung generiert werden. Die Fertigungsdaten können aber auch bereits solche Steuersignale enthalten oder aus solchen Steuersignalen bestehen. Beispielsweise können die Fertigungsdaten auch ein Computermodelle sein, das dann in eine sogenannte Slicer-Software geladen wird, die das Modell zur Ansteuerung eines 3D-Drucker in einzelne Schichten aufteilt.

Unter einem automatisierten Fertigungsverfahren unter Anwendung einer automatisierten Fertigungsanlage wird insbesondere ein Verfahren verstanden, bei dem die orthopädietechnische Einrichtung ohne Zwischenschaltung menschlicher Verstandestätigkeit und weitgehend ohne manuellen Eingriff die orthopädietechnische Einrichtung herstellt. Ein solches automatisiertes Fertigungsverfahren kann beispielsweise ein additives oder generatives Fertigungsverfahren sein, wie beispielsweise ein 3D-Druckverfahren. Aber auch subtraktive Verfahren wie etwa CNC-Fräsen sind denkbar. Solche automatisierten Fertigungsverfahren können dabei auch unter dem Begriff "Rapid Manufacturing Prozess" zusammengefasst werden.

Eine orthopädietechnische Einrichtung im Sinne der vorliegenden Erfindung kann dabei, wie bereits oben kurz angedeutet, eine Orthese oder Prothese oder ein Orthesen- oder Prothesenteil sein. Eine Orthese als orthopädietechnische Einrichtung kann dabei beispielsweise eine Fußorthese, Handorthese, Knieorthese, Rumpforthese oder Kopforthese sein. Eine Prothese als orthopädietechnische Einrichtung kann dabei bspw.auch eine Knieprothese, Armprothese, ein Prothesenschaft, eine Prothesenkosmetik, ein Prothesenfuß oder eine Prothesenhand sein. Diese Aufzählung ist jedoch nicht abschließend zu verstehen.

Auch Exoskelette, die außen an einem Körperteil oder dem gesamten Körper des Trägers angeordnet werden und vom Körper selbst nicht mehr durchzuführende Bewegungen und/oder Tätigkeiten ermöglichen sollen oder den Träger bei Bewegungen oder Tätigkeiten unterstützen sollen, sind orthopädietechnische Einrichtungen im Sinne dieser Erfindung. Dazu zählen auch Vorrichtungen, die es dem Träger erleichtern, kraftaufwendige, anstrengende oder ermüdende Tätigkeiten, beispielsweise Über-Kopfarbeiten, besser, leichter und länger auszuführen.

Erfindungsgemäß ist nun vorgesehen, dass zunächst ein digitales 3D-Körperteilmodell eines Körperteils in einer Datenverarbeitungsanlage bereitgestellt wird. Des Weiteren wird mindestens ein digitales Funktionskomponentenmodell in der Datenverarbeitungsanlage bereitgestellt, wobei das digitale Funktionskomponentenmodell eine orthopädietechnische Funktionskomponente digital abbildet, die in eine orthopädietechnische Einrichtung integrierbar ist. Jedes der digitalen Funktionskomponentenmodelle enthält dabei entsprechende Komponenteneigenschaften der jeweiligen orthopädietechnischen Funktionskomponente, sodass ganz oder teilweise die orthopädietechnischen Funktionskomponenten durch ihre jeweiligen Funktionskomponentenmodelle und den darin enthaltenen Komponenteneigenschaften beschrieben werden. Eine orthopädietechnische Funktionskomponente erweitert dabei den Funktionsumfang der orthopädietechnischen Einrichtung und kann bestimmte Funktionen dem Träger der orthopädietechnischen Einrichtung bereitstellen. Die Funktionskomponente kann aber auch die Funktion oder die gesamte Funktionalität erst bereitstellen, wie bspw. bei einer Prothesenkosmetik als orthopädietechnische Einrichtung, die erst in Verbindung mit einem Kniegelenk mit Fuß die Funktion bereitstellt. Dabei ist es denkbar und auch vorteilhaft, wenn eine Mehrzahl von digitalen Funktionskomponentenmodellen für jeweils verschiedene orthopädietechnische Funktionskomponenten bereitgestellt wird. Dabei kann für eine orthopädietechnische Einrichtung ein oder mehrere Funktionskomponentenodelle bereitgestellt werden.

Es wird nun eine digitale Komponentenschnittstelle mittels der Datenverarbeitungsanlage generiert, die dazu vorgesehen ist, eine ausgewählte Funktionskomponente an oder in die orthopädietechnische Einrichtung zu integrieren. Die digitale Komponentenschnittstelle wird dabei in Abhängigkeit von Komponenteneigenschaften mindestens eines ausgewählten digitalen Funktionskomponentenmodells einer orthopädietechnischen Funktionskomponente, die in die orthopädietechnische Einrichtung integriert werden soll, generiert, wobei die digitale Komponentenschnittstelle eine Aufnahme zum Anordnen der mindestens einen ausgewählten orthopädietechnischen Funktionskomponente aufweist. Bei der Aufnahme kann es sich bspw. um Ausnehmungen, Befestigungseinrichtungen und/oder Abstützungen handeln, die geeignet sind, eine Verbindung zwischen der orthopädietechnischen Einrichtung und der jeweiligen Funktionskomponente herzustellen.

Anschließend wird mittels der Datenverarbeitungsanlage ein digitales orthopädietechnisches Modell der zu fertigenden orthopädietechnischen Einrichtung basierend auf dem 3D-Körperteilmodell und der digitalen Komponentenschnittstelle zur Integration der orthopädietechnischen Funktionskomponente generiert, wodurch die herzustellende orthopädietechnische Einrichtung digital soweit definiert wird, das bis auf die Integration der jeweiligen Funktionskomponente sämtliche Eigenschaften vorhanden sind. Selbstverständlich ist es auch möglich, dass ein Nutzer sowohl vor als auch nach der Generierung des digitalen orthopädietechnischen Modells Anpassungen an diesem oder den zugrundeliegenden Daten manuell oder automatisiert vornimmt.

Schließlich können die digitalen Fertigungsdaten aus dem erstellten digitalen orthopädietechnischen Modell mittels der Datenverarbeitungsanlage generiert werden, um so unter Zuführung der digitalen Fertigungsdaten zu einer digitalen Fertigungsanlage die orthopädietechnische Einrichtung automatisiert so herzustellen, dass anschließend nur noch die Funktionskomponenten mittels der Komponentenschnittstelle und deren Aufnahme integriert werden brauchen.

Damit wird es möglich, orthopädietechnische Einrichtungen weitestgehend automatisiert herzustellen, wobei Funktionskomponenten, die in die orthopädietechnische Einrichtung integriert werden müssen oder sollen, zu einem späteren Zeitpunkt nahtlos in die hergestellten orthopädietechnischen Einrichtung integriert werden können.

Durch die Definition der Funktionskomponenten in Form von digitalen Funktionskomponentenmodellen können sämtliche Eigenschaften dieser Funktionskomponenten beschrieben werden, sodass bei der Erstellung der digitalen Komponentenschnittstelle auf diese Eigenschaften der Funktionskomponenten Rücksicht genommen werden kann.

Das erstellte digitale orthopädietechnische Modell ist dabei insbesondere ein digitales 3-dimensionales Modell, welches die Form, Geometrie, Abmessungen und Eigenschaften der herzustellenden orthopädietechnischen Einrichtung enthält bzw. beschreibt.

Bei dem bereitgestellten 3D-Körperteilmodell kann es sich bspw. um ein Modell eines Körperteils handeln, an das eine orthopädietechnische Einrichtung angebracht werden soll (bspw. eine Orthese oder eine Prothese). Bei dem 3D-Körperteilmodell kann es sich aber auch um ein Modell eines kontralateralen Körperteils handeln, welches als Grundlage für die Nachbildung der jeweils anderen Körperteilseite dient. Dies ist bspw. dann der Fall, wenn mittels einer Prothesenkosmetik ein noch vorhandenes, kontralaterales Körperteil nachgebildet werden soll.

Gemäß einer Ausführungsform ist vorgesehen, dass das 3D-Körperteilmodell ein digitales Abbild des Körperteils ist, für das die orthopädietechnische Einrichtung bestimmt ist, welches in eine orthopädietechnische Zweckform überführt wurde. Das 3D-Körperteilmodell kann somit nicht nur ein digitales Abbild des Körperteils sein, für das die orthopädietechnische Einrichtung bestimmt ist, sondern auch eine orthopädietechnische Zweckform beinhalten oder darstellen, die durch eine medizinische Indikation indiziert ist und/oder individuelle anatomische Gegebenheiten berücksichtigt. Darüber hinaus bietet die Zweckform dem Orthopädietechniker die Möglichkeit, eigene Anpassungen an dem Abbild vorzunehmen und so eigene Erfahrungswerte einfließen zu lassen. So kann bspw. der Schaft so geformt werden, dass knöcherne Strukturen und Weichteile unterschiedliche Druckbelastungen abbekommen.

Das digitale Abbild kann beispielsweise durch einen digitalen Scan erfolgen oder durch einen Abdruck mittels Formmasse. Auch eine parameterische Erfassung des Körperteils ist denkbar, bei dem der Orthopädietechniker wichtige Maße des Körperteils vermisst und mittels der Datenverarbeitungsanlage das Körperteilmodell erstellt. Alternativ sind aber auch Verfahren wie Stereometrie oder MRT/CT denkbar. Auch ist es denkbar, ein Abbild des kontralateralen Körperteils zu verwenden. Dies ist insbesondere bei der Erstellung von Prothesen oder Prothesenkosmetiken von Vorteil, da diese so an das Erscheinungsbild der noch vorhandenen kontralateralen Seite angepasst werden können. Auch die Verwendung von generischen Körperteilmodellen aus Datenbanken oder Simulationen ist denkbar. Diese können bei Bedarf, bspw. über Parameter wie Größe und Gewicht, an den Patienten angepasst werden.

Gemäß einer Ausführungsform ist vorgesehen, dass das bereitgestellte 3D-Körperteilmodell von dem Orthopädietechniker frei modelliert wurde bzw. frei modelliert werden kann. Das Modellieren kann dabei digital erfolgen oder auch zunächst mittels Gips oder anderer Hilfsmittel erfolgen und anschließend eingescannt werden. Auf diese Weise kann der Orthopädietechniker das Körperteilmodell auch unabhängig von dem tatsächlichen Erscheinungsbild modellieren. Dieses Vorgehen kann insbesondere bei Prothesenkosmetiken vorteilhaft sein, da so das Erscheinungsbild der Kosmetik individuell angepasst werden kann und auf Wunsch auch von dem ursprünglichen Körperteil abweichen kann.

Gemäß einer Ausführungsform ist vorgesehen, dass ein digitales orthopädietechnisches Modell in Form eines Volumenmodells erstellt wird. Ein Volumenmodell weist zu einem zweidimensionalen Modell den Vorteil auf, dass die Wandstärke der orthopädietechnischen Einrichtung sichtbar wird.

Gemäß einer Ausführungsform ist vorgesehen, dass zum Generieren einer digitalen mechanischen Schnittstelle eine digitale mechanische Schnittstelle aus einer Mehrzahl von bereitgestellten digitalen mechanischen Schnittstellen in Abhängigkeit von Komponenteneigenschaften des mindestens einen ausgewählten digitalen Funktionskomponentenmodells der orthopädietechnischen Einrichtung ausgewählt wird, dessen Aufnahme der mindestens einen ausgewählten orthopädietechnischen Funktionskomponente entspricht. Demnach ist ergänzend vorgesehen, dass zunächst eine Vielzahl von digitalen mechanischen Schnittstellen bereitgestellt werden, aus denen dann diejenige mechanische Schnittstelle ausgewählt wird, die zu der ausgewählten Funktionskomponente basierend auf den Komponenteneigenschaften passt. Somit wird die mechanische Schnittstelle wie auch die Funktionskomponente aus einer Bibliothek von bereits vorgefertigten mechanischen Schnittstellen ausgewählt, sodass auf mehr oder weniger standardisierte Schnittstellen zurückgegriffen werden kann.

Gemäß einer Ausführungsform ist vorgesehen, dass die digitale Komponentenschnittstelle weiterhin in Abhängigkeit von dem 3D-Körperteilmodell und/oder von einem aus dem 3D-Körperteilmodell erstellten digitalen Modell der zu fertigenden orthopädietechnischen Einrichtung generiert wird.

Gemäß einer Ausführungsform ist vorgesehen, dass mittels der Datenverarbeitungsanlage automatisch ein erstes digitales orthopädietechnisches Teilmodell der zu fertigenden orthopädietechnischen Einrichtung basierend auf dem 3D-Körperteilmodell erstellt wird und ein zweites digitales orthopädietechnisches Teilmodell der zu fertigenden orthopädietechnischen Einrichtung basierend auf der generierten digitalen Komponentenschnittstelle erstellt wird, wobei das digitale orthopädietechnische Modell der zu fertigenden orthopädietechnischen Einrichtung in Abhängigkeit von dem ersten digitalen orthopädietechnischen Modell und dem zweiten digitalen orthopädietechnischen Modell erstellt wird.

Es werden demnach mindestens zwei orthopädietechnische Teilmodell erstellt, die zum einen an das 3D-Körperteilmodell angepasst ist und zum anderen die mechanische Schnittstelle abbildet, wobei beim Erstellen des digitalen orthopädietechnischen Modells dann die beiden Teilmodell zusammengefügt werden, um ein gemeinsames Modell zu generieren, bei dem die mechanische Schnittstelle in die orthopädietechnische Einrichtung integriert ist. Die Erstellung des zweiten digitalen orthopädietechnischen Teilmodells umfasst daher nicht nur das Vorsehen der Aufnahme für die Funktionskomponente, sondern kann auch eine weitere Aufnahme umfassen, mit der die mechanische Schnittstelle an das erste Teilmodell angeordnet wird, um das vollständige Modell zu erhalten. Diese zweite Aufnahme ist dabei abhängig von dem ersten Teilmodell.

Es ist daher besonders vorteilhaft, wenn das zweite digitale orthopädietechnische Modell der zu fertigenden orthopädietechnischen Einrichtung weiterhin in Abhängigkeit von dem zuvor erstellten ersten digitalen orthopädietechnischen Modell erstellt wird.

Gemäß einer Ausführungsform ist vorgesehen, dass für jedes digitalen Funktionskomponentenmodellen einer orthopädietechnischen Funktionskomponente als Komponenteneigenschaft die Maße der Komponente, ein Bauraum der Komponente, ein Bewegungsraum der Komponente, Stabilitätseigenschaften, Zugänge für den Ein- und/oder Ausbau, Werkzeugansätze, Versorgungsleitungen, Entsorgungsleitungen, tolerierbare Momente, entstehende Momente, mögliche Funktionskomponentenkombinationen und/oder thermische Eigenschaften hinterlegt sind. Funktionskomponentenkombinationen sind insofern dann notwendig, mehr als eine Funktionskomponente integriert werden soll, da nicht jede Funktionskomponente auch mit jeder anderen Funktionskomponente technisch harmoniert. Durch das hinterlegen einer Matrix von Funktionskomponentenkombinationen wird es möglich, nur sinnvolle Kombinationen zur Verfügung zu stellen.

Gemäß einer Ausführungsform ist vorgesehen, dass ein oder mehrere Komponenteneigenschaften auf einer Wiedergabeeinheit der Datenverarbeitungsanlage mit oder ohne dem digitalen orthopädietechnischen Modell oder einem Teilmodell hiervon visualisiert werden. Durch das Visualisieren der Komponenteneigenschaften können beispielsweise Bewegungen der Funktionskomponente dargestellt werden, wodurch ersichtlich wird, ob das generierte orthopädietechnische Modell die Randbedingungen an die Funktion der Funktionskomponente erfüllt.

Gemäß einer Ausführungsform ist vorgesehen, dass in Abhängigkeit von mindestens einer Komponenteneigenschaft mindestens einer ausgewählten Funktionskomponente mittels der Datenverarbeitungsanlage überprüft wird, ob die Kombination von ausgewählter Funktionskomponente und bereitgestelltem 3D-Körperteilmodell, einer davon abgeleiteten orthopädietechnischen Zweckform und/oder digitalem orthopädietechnischen Modell oder Teilmodell herstellbar und/oder funktionabel ist. Funktionabel meint insbesondere hierbei, dass die orthopädietechnische Einrichtung zusammen mit der Funktionkompoente als Gesamtsystem die gewünschten Bewegungen durchführen und/oder die erwarteten Belastungen aushalten kann.

Hierdurch wird es außerdem möglich, automatisch durch die Datenverarbeitungsanlage überprüfen zu lassen, ob die Kombination von ausgewählten Funktionskomponenten und dem Körperteilmodell, einer daraus abgeleiteten Zweckform oder dem bereits erstellten orthopädietechnischen Modell oder Teilmodell überhaupt herstellbar ist bzw. technisch funktioniert und die vorgesehenen Funktionen der Funktionskomponenten realisiert werden können. So lässt sich überprüfen, ob der Bewegungsraum vorhanden ist, damit die durch eine Funktionskomponente spezifizierte Bewegung ausführbar ist. Auch lässt sich überprüfen, ob Zugänge so gewählt sind, dass Techniker Zugriff auf Versorgungsleitungen oder andere Funktionskomponenten haben. Es lässt sich außerdem prüfen, ob die erwarteten Belastungen ausgehalten und/oder abgetragen werden können.

Gemäß einer Ausführungsform ist vorgesehen, dass in Abhängigkeit von mindestens einer Komponenteneigenschaft mindestens einer ausgewählten Funktionskomponente mittels der Datenverarbeitungsanlage die Bewegung und/oder die Belastung der herzustellenden orthopädietechnischen Einrichtung und/oder der ausgewählten Funktionskomponente simuliert werden. Durch eine umfangreiche Simulation können dabei die Funktionen der integrierten Funktionskomponenten digital getestet werden.

Gemäß einer Ausführungsform ist vorgesehen, dass ein digitales Oberflächenmodell der zu fertigenden orthopädietechnischen Einrichtung basierend auf dem 3D-Körperteilmodell und einer auf dem 3D-Körperteilmodell vorgegebenen Randbegrenzung der orthopädietechnischen Einrichtung mittels der Datenverarbeitungsanlage erstellt wird, wobei das Oberflächenmodell die Innenseite der späteren orthopädietechnischen Einrichtung bildet und wobei das digitale orthopädietechnische Modell der zu fertigenden orthopädietechnischen Einrichtung basierend auf dem digitalen Oberflächenmodell und einer vorgegebenen Materialstärke der zu fertigenden orthopädietechnischen Einrichtung mittels der Datenverarbeitungsanlage automatisch erstellt wird.

Gemäß einer Ausführungsform ist vorgesehen, dass die orthopädietechnische Einrichtung eine Orthese, insbesondere eine Fußorthese, Handorthese, Knieorthese, Rumpforthese oder Kopforthese ist, eine Prothese oder ein Exoskelett ist.

Die Aufgabe wird im Übrigen auch mit dem Computerprogramm gemäß Anspruch 14 zur Durchführung des vorstehend genannten Verfahrens zum Erstellen von Fertigungsdaten gelöst, wenn das Computerprogramm auf einer Datenverarbeitungsanlage ausgeführt wird. Das Computerprogramm kann dabei vorteilhafterweise auf einem Datenträger gespeichert sein.

Die Aufgabe wird im Übrigen auch mit dem Verfahren zur Fertigung einer orthopädietechnischen Einrichtung gemäß Anspruch 15 erfindungsgemäß gelöst, wobei zunächst die Fertigungsdaten für die orthopädietechnische Einrichtung mit dem Verfahren wie vorstehend beschrieben erstellt werden. Anschließend werden diese Fertigungsdaten einer automatisierten Fertigungsanlage zugeführt, die eingerichtet ist, unter Anwendung der erstellten Fertigungsdaten in einem automatisierten Fertigungsverfahren die orthopädietechnische Einrichtung herzustellen. Eine solche Fertigungsanlage kann beispielsweise ein 3D-Drucker sein. Derartige Fertigungsanlagen sind beispielsweise unter dem Begriff der additiven bzw. generativen Fertigungsanlagen bekannt. Durch das Zuführen der Fertigungsdaten zu der automatisierten Fertigungsanlage wird dann die orthopädietechnische Einrichtung durch das automatisierte Fertigungsverfahren in Abhängigkeit von zugeführten Fertigungsdaten hergestellt.

Bei der beschriebenen Datenverarbeitungsanlage kann es sich um ein einzelnes Gerät handeln, dass durch ein oder mehrere Person gleichzeitig oder nacheinander bedient wird. Denkbar ist aber auch, dass die Datenverarbeitungsanlage über mehrere Rechnersysteme verteilt ist, sodass hier beispielsweise mehrere Personen an verschiedenen Orten Zugriff haben. So ist es, denkbar, dass die Erstellung des 3D-Körperteilmodells durch einen ersten Teil der Datenverarbeitungsanlage generiert wird, während die Erstellung des orthopädietechnischen Modells durch einen zweiten Teil der Datenverarbeitungsanlage erfolgt. Das Erzeugen der Fertigungsdaten kann dann beispielsweise durch einen dritten Teil der Datenverarbeitungsanlage erfolgen. Der erste, zweite und dritte Teil der Datenverarbeitungsanlage müssen dabei nicht zwingend durch eine dasselbe Gerät abgebildet werden, sondern können an verschiedenen Orten durch jeweils eigenständige separate Geräte (Recheneinheiten) realisiert werden.

Die Erfindung wird anhand der beigefügten Figuren beispielhaft näher erläutert. Es zeigen:
- Figur 1: schematische Darstellung des erfindungsgemäßen Verfahrensablaufes;
- Figur 2: Darstellung eines 3D-Körperteilmodells;
- Figur 3: Darstellung eines orthopädietechnischen 3D-Modells;
- Figur 4: Darstellung einer Prothesenkosmetik beim Einsetzen;
- Figur 5: Darstellung der Integration eines ersten Funktionselementes;
- Figur 6: Darstellung eines Innenraumes einer Prothesenkosmetik;
- Figur 7: Darstellung einer Integration eines zweiten Funktionselementes.

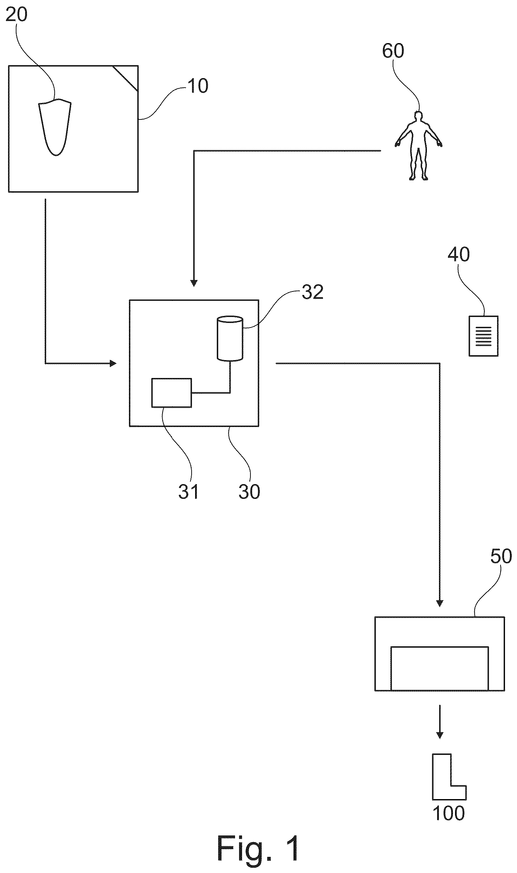

Figur 1 zeigt in einer schematischen Darstellung den erfindungsgemäßen Verfahrensablauf, der mit der Bereitstellung eines 3D-Körperteilmodells 20 in Form einer digitalen Zweckform 10 beginnt. Das 3D-Körperteilmodell 20 ist eine 3-dimensionale Abbildung des betreffenden Körperteils der gehandicapten Person. Im Ausführungsbeispiel der Figur 1 handelt es sich bei der digitalen Zweckform 10 um einen Amputationsstumpf eines amputierten Beines, für den eine Prothese angefertigt werden soll.

Im Idealfall weist die digitale Zweckform bereits eine Form und Geometrie auf, mit der die medizinische Indikation der gehandicapten Person als Maßnahme behandelt werden soll. Die digitale Zweckform weist demzufolge eine Form und Geometrie auf, die dann zu einer entsprechenden Form und Geometrie der Orthese führt, mit der sich die medizinische Indikation der gehandicapten Person behandeln lässt und somit die geeignete Behandlungsmaßnahmen darstellt. Alternativ weist die digitale Zweckform eine an die individuelle Anatomie der gehandicapten Person angepasste Form auf, die dann zu einer entsprechenden Form und Geometrie der Orthese oder Prothese führt, mit der diese besonders gut auf die Form des Körperteils abgestimmt ist und somit zu einem erhöhten Tragekomfort führt. Insbesondere bei Prothesenschäften ist es regelmäßig von Vorteil das 3D-Körperteilmodell zu modifizieren und so eine Zweckform zu erstellen, die der Verteilung von Weichteilgewebe, Muskeln und Knochen Rechnung trägt.

Die digitale Zweckform 10 mit dem 3D-Körperteilmodell 20 wird nun einer Datenverarbeitungsanlage 30 bereitgestellt, die eine Recheneinheit 31 und einen Datenspeicher 32 hat. Recheneinheit und Datenspeicher können dabei auch Cloud-basierte Lösungen sein. In dem Datenspeicher 32 wird eine Mehrzahl von digitalen Funktionskomponentenmodellen bereitgestellt, die jeweils in die herzustellende orthopädietechnische Einrichtung integrierbar sind und eine spezielle, zusätzliche Funktion für die orthopädietechnische Einrichtung schaffen bzw. bereitstellen.

Im ersten Schritt ist die Recheneinheit 31 im Ausführungsbeispiel der Figur 1 nun so ausgebildet, dass sie basierend auf dem 3D-Körperteilmodell 20 oder der digitalen Zweckform 10 eine Analyse durchführt, um so ein erstes digitales orthopädietechnisches Modell der zu fertigenden orthopädietechnischen Einrichtung zu erstellen.

Ein Techniker 60 kann dabei Einfluss auf das erste Teilmodell nehmen. Der Techniker 60 hat des Weiteren die Möglichkeit, ein oder mehrere orthopädietechnische Funktionskomponenten auszuwählen, die in die herzustellende orthopädietechnische Einrichtung integriert werden sollen. Basierend auf der Auswahl des Technikers 60 werden dann die digitalen Funktionskomponentenmodelle aus dem Datenspeicher 32 einschließlich der für jedes digitale Funktionskomponentenmodell hinterlegten Komponenteneigenschaften abgerufen.

Im nächsten Schritt ist die Recheneinheit 31 im Ausführungsbeispiel der Figur 1 und so ausgebildet, dass basierend auf den Komponenteneigenschaften mindestens eines ausgewählten digitalen Funktionskomponentenmodells einer orthopädietechnischen Funktionskomponente eine digitale Komponentenschnittstelle generiert wird, aus der dann ein zweites digitales orthopädietechnisches Teilmodell erstellt wird. Die digitale Komponentenschnittstelle weist dabei eine Aufnahme auf, die individuell an die ausgewählte Funktionskomponente basierend auf den Komponenteneigenschaften und dem digitalen Funktionskomponentenmodell so angepasst ist, dass sich die Funktionskomponente problemlos in die digitale Komponentenschnittstelle integrieren lässt.

Im Bereich der Aufnahme ist die digitale Komponentenschnittstelle dabei an die ausgewählte Funktionskomponente entsprechend den Komponenteneigenschaften angepasst, um die Funktionskomponente später problemlos aufnehmen zu können.

Aus dem ersten digitalen orthopädietechnischen Teilmodell und dem zweiten digitalen orthopädietechnische Teilmodell wird nun mittels der Recheneinheit 31 ein digitales orthopädietechnisches Modell der orthopädietechnischen Einrichtung erstellt, indem die beiden Teilmodelle zu einem gemeinsamen Gesamtmodell zusammengeführt werden. Die digitale Komponentenschnittstelle wird dabei in dem Bereich, in dem das zweite Teilmodell dem ersten Teilmodell verschmolzen wird, vorzugsweise so angepasst, dass das zweite Teilmodell in diesem Bereich dem ersten Teilmodell im Wesentlichen entspricht.

Nach dem Erstellen des digitalen orthopädietechnischen Modells werden dann entsprechende Fertigungsdaten 40 generiert, die dann an eine automatisierte Fertigungsanlage 50 übertragen werden. Bei den Fertigungsdaten 40 kann es sich im einfachsten Fall um das erstellte digitale Modell handeln, welches dann von der automatisierten Fertigungsanlage 50 zur Ansteuerung der Anlage analysiert wird, um die entsprechenden Steuersignale zur Ansteuerung der automatisierten Fertigungsanlage 50 zu erzeugen. Denkbar ist aber auch, dass die Fertigungsdaten 40 bereits jene Steuersignale enthalten, die zur Ansteuerung der automatisierten Fertigungsanlage 50 dienen. Letztlich hängt dies vom konkreten Anwendungsfall ab und der Art der automatisierten Fertigungsanlage 50 bzw. das von der Fertigungsanlage 50 ausgeführten automatisierten Fertigungsverfahren.

Im Ausführungsbeispiel der Figur 1 handelt es sich bei der automatisierten Fertigungsanlage 50 um eine 3D-Druckanlage, mit der in einem additiven oder generativen Fertigungsverfahren die Prothese 100 basierend auf dem digitalen Modell automatisch hergestellt werden kann. Nach der Herstellung der Prothese 100 können dann in die in der Komponentenschnittstelle vorgesehenen Aufnahmen der jeweiligen Funktionskomponente physisch in die Prothese 100 eingesetzt werden. Bei der Prothese 100 kann es sich dabei beispielsweise um einen Prothesenschaft handeln, an dem noch zusätzliche Prothesenelemente angeordnet werden müssen.

Die Datenverarbeitungsanlage 30 ist darüber hinaus eingerichtet, einen manuellen Eingriff durch einen Techniker 60 zu gewähren, um so die Möglichkeit zu schaffen, das Modell und somit die herzustellende orthopädietechnische Einrichtung manuell zu manipulieren. Hierdurch können Sonderwünsche berücksichtigt werden, die automatisiert nicht herstellbar sind.

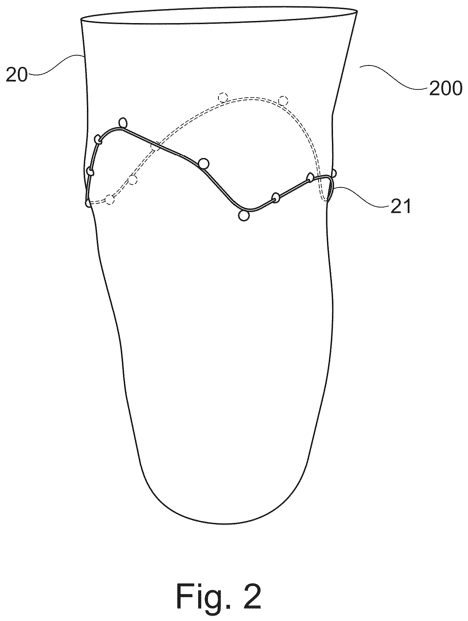

Figur 2 zeigt einen Amputationsstumpf 200 eines amputierten Beines, an dem eine Prothese angeordnet werden soll. Der in Figur 2 gezeigt der Amputationsstumpf 200 ist dabei ein 3D-Körperteilmodell 20, welches die Grundlage für die Erstellung des Prothesenschafts ist. Das 3D-Körperteilmodell 20 wurde dabei durch einen Techniker bereits dahingehend ergänzt, dass Randbegrenzungen 21 vorgesehen sind, welche den oberen Abschluss des Prothesenschafts darstellen.

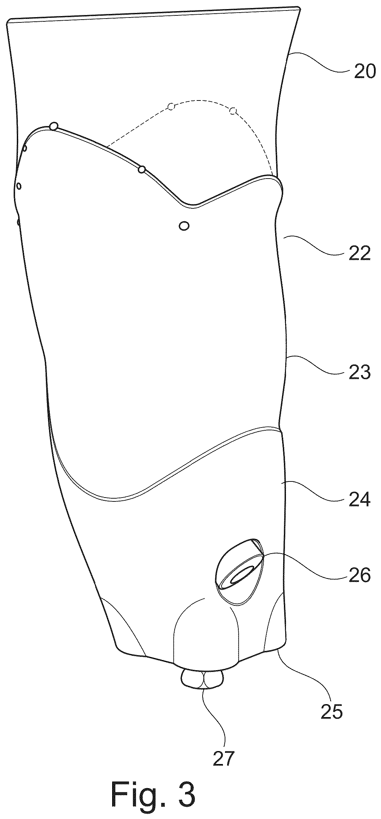

Figur 3 zeigt das fertige digitale orthopädietechnische Modell 22, wie es an dem 3D-Körperteilmodell 20 angeordnet ist. Das digitale Modell 22 weist dabei ein erstes digitales Teilmodell 23 und ein zweites digitales Teilmodell 24 auf, die zu einem gemeinsamen digitalen Modell 22 verschmolzen sind. Das zweite digitale Teilmodell 24 bildet dabei die digitale Komponentenschnittstelle 25, mit der Funktionskomponenten 26, 27 in die orthopädietechnische Einrichtung insgesamt integriert werden sollen.

Dieses digitale Modell 22 kann nun als Grundlage zur Generierung von digitalen Fertigungsdaten verwendet werden, um so auf dieser Basis dann eine orthopädietechnische Einrichtung herstellen zu lassen, die dann später die Funktionskomponente entsprechend in die vorgesehenen Aufnahmen eingesetzt werden.

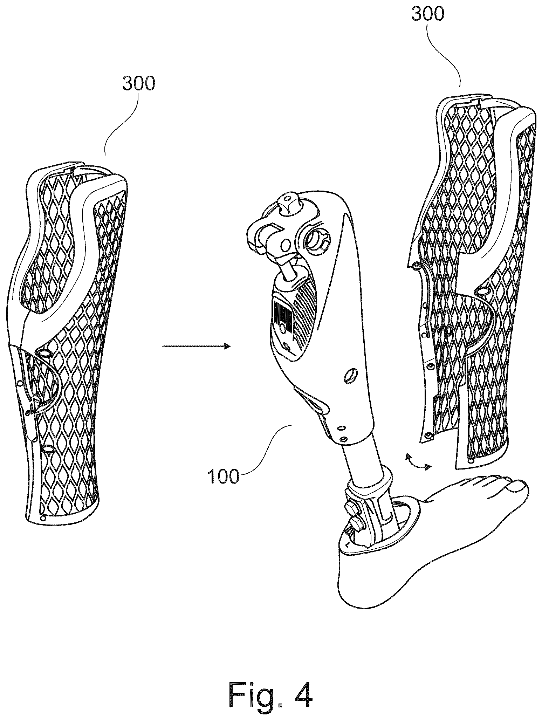

Figur 4 zeigt eine Prothesenkosmetik 300 als eine orthopädietechnische Einrichtung, wobei die Prothesenkosmetik 300 an ein mechatronisches Kniegelenk 100 als Funktionskomponente angeordnet werden soll. Die Funktionskomponente erlaubt hier beispielsweise eine gesteuerte Dämpfung der Kniebewegung. Prothesenkosmetik 300 und Funktionskomponente 100 bilden zusammen das Prothesenknie. Distal an dem Prothesenknie angeordnet befindet sich ein Prothesenfuß als weitere Funktionskomponente. Auch für diese kann die Prothesenkosmetik 300 eine Schnittstelle vorsehen

Die Prothesenkosmetik 300 dient dabei dazu, das Erscheinungsbild der Prothese 100 zu verbessern und insbesondere das Erscheinungsbild an ein noch vorhandenes kontralaterales Körperteil, hier ein Fuß mit Bein, anzupassen. Weiterhin stellt die Prothesenkosmetik eine Schutzwirkung für die Funktionskomponenten bereit.

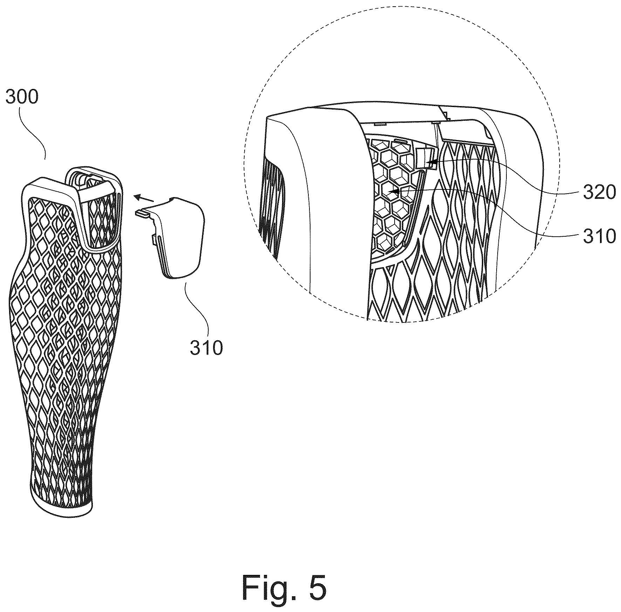

Wie in Figur 5 gezeigt, können in eine solche Prothesenkosmetik 300 auch weitere Funktionskomponente 310 integriert werden, um die Funktion der Prothesenkosmetik 300 weiter zu verbessern. Im Ausführungsbeispiel der Figur 5 wird dabei in die Prothesenkosmetik 300 eine Funktionskomponente 310 in Form eines Softknies eingesetzt, um so beim Abstützen oder beim Hinknien die Prothesenkosmetik 300 nicht zu beschädigen. Die Prothesenkosmetik 300 weist hierfür eine Schnittstelle 320 auf, in die die Funktionskomponente 310 eingesetzt wird. Im Ausführungsbeispiel der Figur 5 wird die Funktionskomponente 310 hierbei rastend eingesetzt und gehalten.

Beim Generieren der digitalen Komponentenschnittstelle ist bei Auswahl der in Figur 5 gezeigten Funktionskomponente 310 eine entsprechende Rastaufnahme vorzusehen, in die die Funktionskomponente 310 ein gerastet werden kann.

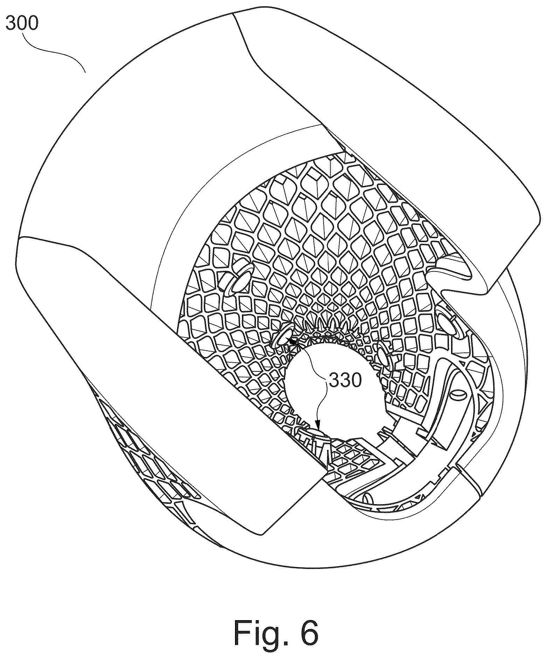

Figur 6 zeigt eine Darstellung einer aus der Figur 4 und 5 bekannten Prothesenkosmetik 300 mit einer inneren Sicht. Im Inneren der Prothesenkosmetik 300 ist dabei eine zweite Komponentenschnittstelle 330 vorgesehen, die eine Aufnahme zum Anordnen einer orthopädietechnischen Funktionskomponente in Form einer Prothese bilden. Durch das Schließen der Prothesenkosmetik 300 drückt diese zweite Schnittstelle 320 als elastische Elemente gegen die umschließende Prothese (nicht dargestellt), sodass die Prothesenkosmetik 300 so gehalten wird.

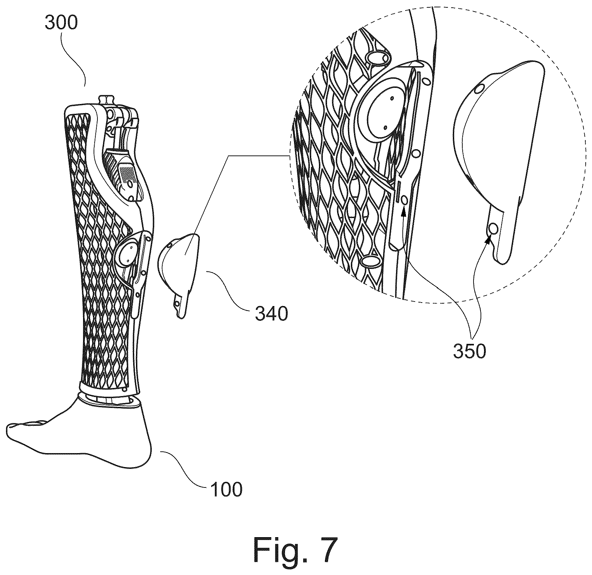

Figur 7 zeigt in einer weiteren Ausführungsform eine Prothesenkosmetik 300, in die eine weitere, dritte Funktionskomponente 340 eingesetzt bzw. einsetzbar ist. Bei der in Figur 7 dargestellten Funktionskomponente 340 handelt es sich um eine Abdeckung, mit der eine dahinter liegende und in der abgedeckten Prothese vorhandene Funktionseinheit geschützt werden soll.

In der Prothesenkosmetik 300 ist dabei eine Öffnung vorgesehen, in die im Randbereich Magnete eingebracht sind, die zusammen mit der Öffnung eine dritte Schnittstelle 350 bilden, um so die dritte Funktionskomponente 340 (Abdeckung) aufnehmen zu können. In der dritten Funktionskomponente 340 sind dabei ebenfalls Magnete eingebracht, die mit den Magneten in der Prothesenkosmetik 300 so zusammenwirken, dass die dritte Funktionskomponente 340 magnetisch in der Öffnung gehalten wird. Die Abdeckung stellt dabei den Schutz der Funktionskomponente sicher, ermöglicht aber auch den schnellen und einfachen Zugang zu dieser. Im dargestellten Beispiel wird so der einfache Zugang zum Ladeanschluss der Funktionskomponente ermöglicht.

Auch weitere Aspekte der Kosmetik, wie etwa die Wabenstruktur, können auf den Bedarf der jeweils ausgewählten Funktionskomponenten angepasst werden. Eine Struktur mit vielen und großen Öffnungen kann beispielsweise für hydraulische Kniegelenke vorgesehen werden, um so einen ausreichenden Abtransport der bei der Dämpfung entstehenden Wärme zu ermöglichen.

### Bezugszeichenliste

- 10: digitale Zweckform
- 20: 3D-Körperteilmodell
- 21: Randbegrenzung
- 22: digitales orthopädietechnisches Modell
- 23: erstes digitales Teilmodell
- 24: zweites digitales Teilmodell
- 25: digitale Komponentenschnittstelle
- 26: Funktionskomponente
- 27: Funktionskomponente
- 30: Datenverarbeitungsanlage
- 31: Recheneinheit
- 32: Datenspeicher
- 40: Fertigungsdaten
- 50: Fertigungsanlage
- 100: Prothese
- 200: Amputationsstumpf
- 300: Prothesenkosmetik
- 310: zweite Funktionskomponente
- 320: erste Schnittstelle
- 330: zweite Schnittstelle
- 340: dritte Funktionskomponente
- 350: dritte Schnittstelle

## Patentansprüche

1. Verfahren zum Erstellen von Fertigungsdaten (40) zur Fertigung einer orthopädietechnischen Einrichtung, die unter Anwendung der erstellten Fertigungsdaten (40) in einem automatisierten Fertigungsverfahren herstellbar ist, wobei das Verfahren die folgenden Schritte umfasst:
- Bereitstellen eines digitalen 3D-Körperteilmodells (20) eines Körperteils in einer Datenverarbeitungsanlage (30),
- Bereitstellen von mindestens einem digitalen Funktionskomponentenmodell einer orthopädietechnischen Funktionskomponente (26,27) in der Datenverarbeitungsanlage (30), die in eine orthopädietechnische Einrichtung integrierbar ist, wobei das digitale Funktionskomponentenmodell entsprechende Komponenteneigenschaften der jeweiligen orthopädietechnischen Funktionskomponente (26,27) enthält,
- Generieren mindestens einer digitalen Komponentenschnittstelle (25) mittels der Datenverarbeitungsanlage (30) in Abhängigkeit von mindestens einer Komponenteneigenschaft wenigstens eines ausgewählten digitalen Funktionskomponentenmodells einer orthopädietechnischen Funktionskomponente (26,27), die in die orthopädietechnische Einrichtung integriert werden soll, wobei die digitalen Komponentenschnittstelle (25) eine Aufnahme zum Anordnen der mindestens einen ausgewählten orthopädietechnischen Funktionskomponente (26,27) aufweist,
- Automatisches Erstellen eines digitalen orthopädietechnischen Modells der zu fertigenden orthopädietechnischen Einrichtung basierend auf dem 3D-Körperteilmodell (20) und der digitalen Komponentenschnittstelle (25) zur Integration der orthopädietechnischen Funktionskomponente (26,27) mittels der Datenverarbeitungsanlage (30), und
- Generieren der digitalen Fertigungsdaten (40) aus dem erstellten digitalen orthopädietechnischen Modell mittels der Datenverarbeitungsanlage (30).

2. Verfahren nach Anspruch 1, **dadurch gekennzeichnet, dass** das 3D-Körperteilmodell (20) ein digitales Abbild des Körperteils ist, für das die orthopädietechnische Einrichtung bestimmt ist, welches in eine orthopädietechnische Zweckform überführt wurde.

3. Verfahren nach Anspruch 1 oder 2, **dadurch gekennzeichnet, dass** ein digitales orthopädietechnisches Modell (22) in Form eines Volumenmodells erstellt wird.

4. Verfahren nach einem der vorhergehenden Ansprüche, **dadurch gekennzeichnet, dass** zum Generieren einer digitalen mechanischen Schnittstelle eine digitale mechanische Schnittstelle aus einer Mehrzahl von bereitgestellten digitalen mechanischen Schnittstellen in Abhängigkeit von Komponenteneigenschaften des mindestens einen ausgewählten digitalen Funktionskomponentenmodells der orthopädietechnischen Einrichtung ausgewählt wird, dessen Aufnahme der mindestens einen ausgewählten orthopädietechnischen Funktionskomponente (26,27) entspricht.

5. Verfahren nach einem der vorhergehenden Ansprüche, **dadurch gekennzeichnet, dass** die digitale Komponentenschnittstelle (25) weiterhin in Abhängigkeit von dem 3D-Körperteilmodell (20) und/oder von einem aus dem 3D-Körperteilmodell (20) erstellten digitalen Modell der zu fertigenden orthopädietechnischen Einrichtung generiert wird.

6. Verfahren nach einem der vorhergehenden Ansprüche, **dadurch gekennzeichnet, dass** mittels der Datenverarbeitungsanlage (30) automatisch
- ein erstes digitales orthopädietechnisches Teilmodell (23) der zu fertigenden orthopädietechnischen Einrichtung basierend auf dem 3D-Körperteilmodell (20) erstellt wird, und
- ein zweites digitales orthopädietechnisches Teilmodell (24) der zu fertigenden orthopädietechnischen Einrichtung basierend auf der generierten digitalen Komponentenschnittstelle (25) erstellt wird,
- wobei das digitale orthopädietechnische Modell der zu fertigenden orthopädietechnischen Einrichtung in Abhängigkeit von dem ersten digitalen orthopädietechnischen Modell und dem zweiten digitalen orthopädietechnischen Modell erstellt wird.

7. Verfahren nach Anspruch 6, **dadurch gekennzeichnet, dass** das zweite digitale orthopädietechnische Modell der zu fertigenden orthopädietechnischen Einrichtung weiterhin in Abhängigkeit von dem zuvor erstellten ersten digitalen orthopädietechnischen Modell erstellt wird.

8. Verfahren nach einem der vorhergehenden Ansprüche, **dadurch gekennzeichnet, dass** für jedes digitalen Funktionskomponentenmodellen einer orthopädietechnischen Funktionskomponente (26,27) als Komponenteneigenschaft die Maße der Komponente, ein Bauraum der Komponente, ein Bewegungsraum der Komponente, Stabilitätseigenschaften, Zugänge für den Ein- und/oder Ausbau, Werkzeugansätze, Versorgungsleitungen, Entsorgungsleitungen, tolerierbare Momente, entstehende Momente, mögliche Funktionskomponentenkombinationen und/oder thermische Eigenschaften hinterlegt sind.

9. Verfahren nach einem der vorhergehenden Ansprüche, **dadurch gekennzeichnet, dass** ein oder mehrere Komponenteneigenschaften auf einer Wiedergabeeinheit der Datenverarbeitungsanlage (30) mit oder ohne dem digitalen orthopädietechnischen Modell oder einem Teilmodell hiervon visualisiert werden.

10. Verfahren nach einem der vorhergehenden Ansprüche, **dadurch gekennzeichnet, dass** in Abhängigkeit von mindestens einer Komponenteneigenschaft mindestens einer ausgewählten Funktionskomponente (26,27) mittels der Datenverarbeitungsanlage (30) überprüft wird, ob die Kombination von ausgewählter Funktionskomponente (26,27) und bereitgestelltem 3D-Körperteilmodell (20), einer davon abgeleiteten orthopädietechnischen Zweckform und/oder digitalem orthopädietechnischen Modell oder Teilmodell herstellbar und/oder funktionabel ist.

11. Verfahren nach einem der vorhergehenden Ansprüche, **dadurch gekennzeichnet, dass** in Abhängigkeit von mindestens einer Komponenteneigenschaft mindestens einer ausgewählten Funktionskomponente (26,27) mittels der Datenverarbeitungsanlage (30) die Bewegung und/oder die Belastung der herzustellenden orthopädietechnischen Einrichtung und/oder der ausgewählten Funktionskomponente (26,27) simuliert wird.

12. Verfahren nach einem der vorhergehenden Ansprüche, **dadurch gekennzeichnet, dass** ein digitales Oberflächenmodell der zu fertigenden orthopädietechnischen Einrichtung basierend auf dem 3D-Körperteilmodell (20) und einer auf dem 3D-Körperteilmodell (20) vorgegebenen Randbegrenzung (21) der orthopädietechnischen Einrichtung mittels der Datenverarbeitungsanlage (30) erstellt wird, wobei das Oberflächenmodell die Innenseite der späteren orthopädietechnischen Einrichtung bildet und wobei das digitale orthopädietechnische Modell der zu fertigenden orthopädietechnischen Einrichtung basierend auf dem digitalen Oberflächenmodell und einer vorgegebenen Materialstärke der zu fertigenden orthopädietechnischen Einrichtung mittels der Datenverarbeitungsanlage (30) automatisch erstellt wird.

13. Verfahren nach einem der vorhergehenden Ansprüche, **dadurch gekennzeichnet, dass** die orthopädietechnische Einrichtung eine Orthese, insbesondere eine Fußorthese, Handorthese, Knieorthese, Rumpforthese oder Kopforthese ist, eine Prothese (100) oder ein Exoskelett ist.

14. Computerprogramm mit Programmcodemitteln eingerichtet zur Durchführung des Verfahrens nach einem der vorhergehenden Ansprüche, wenn das Computerprogramm auf einer Datenverarbeitungsanlage (30) ausgeführt wird.

15. Verfahren zur Fertigung einer orthopädietechnischen Einrichtung, wobei das Verfahren die folgenden Schritte umfasst:
- Erstellen von Fertigungsdaten (40) für die orthopädietechnische Einrichtung mit dem Verfahren nach einem der Ansprüche 1 bis 13,
- Zuführen der Fertigungsdaten (40) zu einer automatisierten Fertigungsanlage (50), die unter Anwendung der erstellten Fertigungsdaten (40) in einem automatisierten Fertigungsverfahren die orthopädietechnische Einrichtung herstellt.

16. Verfahren nach Anspruch 15, **gekennzeichnet durch** Herstellen der orthopädietechnischen Einrichtung durch das automatisierte Fertigungsverfahren mittels der automatisierten Fertigungsanlage (50) in Abhängigkeit von den zugeführten Fertigungsdaten (40).

17. Verfahren nach Anspruch 15 oder 16, **dadurch gekennzeichnet, dass** nach der Herstellung der orthopädietechnischen Einrichtung durch das automatisierte Fertigungsverfahren die mindestens eine Funktionskomponente (26,27) an der Aufnahme der Komponentenschnittstelle (25) der orthopädietechnischen Einrichtung befestigt wird.
